# EUROPEAN PATENT SPECIFICATION

(11) **EP 1 474 003 B1**
(45) Date of publication and mention of the grant of the patent: **09.01.2008**
(21) Application number: 03707695.7
(22) Date of filing: 04.02.2003
(51) Int. Cl.: A23C 9/20, A23C 11/04

(54) **HUMAN MILK SUPPLEMENT**
MUTTERMILCHZUSATZ
SUPPLEMENT POUR LE LAIT HUMAIN

(30) Priority: 04.02.2002 US 354240 P
(43) Date of publication of application: 10.11.2004
(73) Proprietor: Bristol-Myers Squibb Company, Princeton, NJ 08543-4000 (US)
(72) Inventor: EUBER, John, Russell, Evansville, IN 47725 (US); HANSEN, James, Wayne, Evansville, IN 47711 (US)
(74) Representative: Duckett, Anthony Joseph
(86) International application number: PCT/US2003/003244
(87) International publication number: WO 2003/065816

(56) References cited:
- EP-A- 0 953 289
- US-A- 5 308 832
- US-B1- 6 294 206
- ROENNHOLM K A R ET AL: "HUMAN MILK PROTEIN AND MEDIUM-CHAIN TRIGLYCERIDE OIL SUPPLEMENTATION OF HUMAN MILK: PLASMA AMINO ACIDS IN VERY LOW-BIRTH-WEIGTH INFANTS" PEDIATRICS, AMERICAN ACADEMY OF PEDIATRICS, EVANSTON, IL,, US, vol. 74, no. 5, 1984, pages 792-799, XP000933587 ISSN: 0031-4005
- BHATIA J ET AL: "HUMAN MILK SUPPLEMENTATION.DELIVERY OF ENERGY, CALCIUM, PHOSPHORUS,MAGNESIUM, COPPER, AND ZINC" AMERICAN JOURNAL OF DISEASES OF CHILDREN, AMERICAN MEDICAL SOCIETY, CHICAGO, IL, US, vol. 142, no. 4, 1988, pages 445-447, XP000929589 ISSN: 0002-922X

## Description

### Field of Invention

The present invention relates to low osmolality nutrient supplements for premature infants and methods to support the rapid growth of premature infants by administering nutritionally supplemented human milk to those infants.

### Background

Pre-term or premature infants are typically infants bom before the 37th week of gestation and/or weighing at birth less than 2500 grams. Many of these infants, because of their developmental immaturity and low weight, present special nutritional needs that normally cannot by met by their mothers' milk or banked human milk. Donor milk, in addition, raises health concerns of potential adulteration with bacteria, viruses or other contaminants.

In general, human milk, because of its nutrient composition and immunological properties, is considered an ideal food for infants. However, human milk is typically too low in proteins and certain minerals such as calcium and phosphorus to meet the needs for rapid growth of many pre-term infants. Protein, a crucial nutrient for infants' growth and synthesis of enzymes and hormones, and certain minerals such as calcium and phosphorus that are needed for appropriate bone development and bone density, must be provided to pre-term infants in the form of human milk nutritional supplements or fortifiers.

Furthermore, the caloric content of human milk typically requires that pre-term infants be fed a volume of milk that is too high to be well tolerated by the infants. Normally, premature infants may tolerate total daily feedings of between 100 to 150 ml per kg of the infants' weight. Since the caloric content of human milk is approximately 67 Kcal per 100 ml of milk (20 Kcal per fluid ounce of milk), and pre-term infants need approximately 120 Kcal per kg of weight per day, human milk can supply only about 80 percent of the infant's energy needs. Thus, to provide a caloric intake that meets the need for pre-term infants to grow rapidly with a volume of milk that the infants can tolerate, the caloric content of the human milk should be supplemented with a source of energy such as carbohydrates, in addition to protein and minerals. For these purposes, nutritional supplements are designed such that, when added to human milk, the supplemented human milk is capable of delivering to the infant approximately 24 Kcal per fluid ounce (approximately 81 Kcal per 100 ml), together with amounts of protein and minerals that are higher than those normally present in human milk.

While the use of nutrient supplements is an attractive solution for the special nutritional needs of pre-term infants, the additional nutrients generally cause an increase in the osmolality of the supplemented human milk over the levels that are typical in human milk. Osmolality refers to the concentration of osmotically-active particles in an aqueous solution per unit weight of solvent, and is expressed in mOsm/kg. When two solutions employing the same solvent but having different osmolality are contacted through a membrane permeable only to the solvent, the solvent will flow from the low osmolality solution to the high osmolality solution. This phenomenon is particularly pronounced when the dissolved compounds are certain species, such as simple carbohydrates and electrolytes, that are known to have high osmotic activity. Other species such as emulsified fats, the form of fat added to nutrient supplements, on the other hand, have low or no osmotic activity. When a hyperosmolar solution, i.e., an aqueous solution having osmolality higher than that of normal body fluids (approximately 300 mOsm/kg of water), is ingested, certain undesirable gastrointestinal side effects may take place. The hyperosmolar solution may cause an osmotic effect in the stomach and small intestine!: water is drawn into the gastrointestinal tract to dilute the concentration of the osmotically-active particles. The influx of water into the gastrointestinal tract may cause diarrhea, nausea, cramping, abdominal distension, regurgitation and vomiting.

Carbohydrates are an energy source readily available for incorporation in nutritional supplements. However, they may have high osmotic activity, particularly simple carbohydrates or those carbohydrates that are highly hydrolyzed. Even complex carbohydrates can detrimentally affect the supplemented human milk osmolality since they may be rapidly hydrolyzed by amylase, an enzyme normally present in human milk. As a result, the osmolality of supplemented human milk may be about 90 to 120 mOsm/kg above normal osmolality levels in unsupplemented milk.

US 5,308,832 describes an enteral nutritional product for a person having a neurological injury, such as from trauma to the head. The product is described as being very low in carbohydrate, but high in fat.

EP-A-0953289 describes a high fat and high protein content milk replacer and process for its production. Preferably the milk replacer composition also contains at least 10% of a carbohydrate by weight.

### Summary of the Invention

The present invention provides the use of: (a) a fat component, and (b) a carbohydrate component, for the preparation of a human milk supplement for prompting the growth of a preterm infant, wherein said fat component is present in said supplement in an amount of 35% by dry weight or more, and said carbohydrate component is present in said supplement in an amount of 10% by dry weight or less.

The present invention also provides a method of supplementing the nutritional value of human milk comprising adding to said human milk a supplement comprising: (a) 35% by dry weight or more of a fat component, and (b) 10% by dry weight or less of a carbohydrate component.

Among the several advantages found to be achieved by the present invention, the substitution of fats for carbohydrates in the nutrient supplement results in a smaller increase in the nutritionally supplemented human milk osmolality and, thus, an increased tolerance to supplemented human milk by premature infants.

### Detailed Description of the Preferred Embodiments

In accordance with the present invention, it has been discovered that the substitution of fats for carbohydrates in nutrient supplements that are added to human milk for administration to premature infants results in a supplemented human milk that has an osmolality closer to that of unsupplemented human milk and which is well tolerated by most premature infants. The nutrient supplement of the present invention (when in powder or liquid form) comprises proteins, fats and carbohydrates in various degrees. However, the present invention requires that fat be at least about 35% by dry weight and that the carbohydrate content be limited to no more than about 10% by dry weight. In this manner, osmolality increases resulting from the additional nutrients are less than about 35 to 40 mOsm/kg

Most any fat can be used in the present invention, provided it is suitable for combination with the other components of the supplement. Exemplary fats include soy oil, medium chain triglycerides (MCT oil), corn oil, sunflower oil, safflower oil, coconut oil, palm oil, cottonseed oil, high oleic safflower, high oleic sunflower, and canola oil. The fat source can comprise one or more of these oils. Emulsifiers, such as lecithin, may replace a small portion of the fat composition, but usually not more than 2%.

Any carbohydrates suitable for infant consumption may be used in the present invention. Commercial sources for these carbohydrates are known to the ordinary practitioner of the art. One particular carbohydrate that could be utilized is corn syrup solids.

Protein sources suitable for use in the present invention include most any protein or nitrogen source suitable for infant consumption. These products are commercially available and their commercial sources are known by practitioners of this art. Both, intact and hydrolyzed proteins, such as hydrolyzed whey protein, can be used. Two particular proteins that can be used are low lactose milk protein isolate (Alapro 9405, from NZMP Co.) and hydrolyzed whey protein isolate (BioZate 3, from Davisco Foods).

Vitamins that may be employed include vitamin A, vitamin D, vitamin E, vitamin K1, thiamin, riboflavin, vitamin B6, vitamin B12, niacin, folic acid, panthotenic acid, biotin, and Vitamin C. Mineral nutrients that may be added include calcium, phosphorus, magnesium, zinc, manganese, copper, sodium, potassium, chloride, and iron. In the present invention as shown in Table 1, these mineral nutrients were added in the form of salts such as calcium phosphate, calcium glycerophosphate, calcium gluconate, sodium citrate, potassium chloride, potassium citrate, potassium phosphate, magnesium phosphate, ferrous sulfate, zinc sulfate, and cupric sulfate. Other vitamins and minerals that can be added are within the knowledge of the person with ordinary skill in the art who can determine the appropriate amount of vitamins and mineral nutrients following the recommendations of the Committee on Nutrition of the American Academy of Pediatrics or other groups of experts.

The following example describes an embodiment of the invention. Other embodiments within the scope of the claims herein will be apparent to one skilled in the art from consideration of the specification or practice of the invention as disclosed herein. It is intended that the specification, together with the examples, be considered exemplary only. In the example all percentages are given on a dry weight basis unless otherwise indicated.

### Example

This Example demonstrates an embodiment of the composition of the nutritional supplement of this invention. Table 1 shows the amount of base nutrients (proteins, fats and carbohydrates), vitamins and mineral nutrients present in 2.84 grams of a nutritional supplement powder. Actual levels of nutrients may be slightly higher to ensure the indicated levels are delivered over shelf life and for all batches of product. The caloric content of those 2.84 grams of powder is approximately 14 Kcal. Thus, this amount of powder is a recommended dose of nutrient supplement to be added to 100 ml of human milk.

**Table 1**

| **Composition of 2.84 grams of nutritional supplement powder Caloric content: 14 Kcalories** | |
|---|---|
| Caloric Content | |
| Energy, kcal | 14.0 |

| Powder Base | |
|---|---|
| Protein, g | 1.1 |
| Fat, g | 1.0 |
| Carbohydrate, g | 0.23 |
| Ash, g | 0.37 |
| Linoleic Acid, mg | 140 |
| Linolenic Acid, mg | 17 |

| Vitamins | |
|---|---|
| Vitamin A, IU | 950 |
| Vitamin D, IU | 150 |
| Vitamin E, IU | 4.6 |
| Vitamin K₁, mcg | 4.4 |
| Thiamin, mcg | 150 |
| Riboflavin, mcg | 220 |
| Vitamin B₆, mcg | 115 |
| Vitamin B₁₂, mcg | 0.18 |
| Niacin, mcg | 3000 |
| Folic Acid, mcg | 25 |
| Pantothenic Acid, mcg | 730 |
| Biotin, mcg | 2.7 |
| Vitamin C, mg | 12 |

| Minerals | |
|---|---|
| Calcium, mg | 90 |
| Phosphorus, mg | 50 |
| Magnesium, mg | 1 |
| Iron, mg | 1.44 |
| Zinc, mg | 0.72 |
| Manganese, mcg | 10 |
| Copper, mcg | 44 |
| Sodium, mg | 16 |
| Potassium, mg | 29 |
| Chloride, mg | 13 |

This embodiment of the invention can be achieved by adding vitamins and minerals to a powder mix to yield a product containing 39.92% proteins, 36.22% fats, and 8.04% carbohydrates, as illustrated in Table 2 that shows an analysis of 100 grams of nutritional supplement powder.

**Table 2**

| **Analysis per 100 Grams Powder** | |
|---|---|
| Protein, g | 39.92 |
| Fat, g | 36.22 |
| Carbohydrate (by diff.), g | 8.04 |
| Ash, g | 12.89 |
| Moisture, g | 2.93 |

For the above protein, fat, and carbohydrate composition, the caloric distribution is shown in Table 3. In Table 3, it can be seen that 32.3% of the caloric content of the nutritional supplement has a protein source, 62.6% a fat source, and 5.1 % a carbohydrate source.

**Table 3**

| **Caloric Distribution** | |
|---|---|
| Protein | 32.3% |
| Fat | 62.6% |
| Carbohydrate (by diff.) | 5.1% |

In this particular embodiment, the protein sources are low lactose milk protein isolate and hydrolyzed whey protein isolate. Table 4 shows the proportion in which these two protein sources are present in 2.84 grams of powder, the amount of powder that is used as a base to describe in Table 1 the composition.

**Table 4**

| **Protein per 14 Calories (2.84 grams powder)** | |
|---|---|
| Total Protein, g | 1.1 |
| Whey Protein, g | 0.66 |
| Casein Protein, g | 0.44 |

In this particular embodiment, the fat sources are medium chain triglycerides (MCT oil), soybean oil and lecithin. Table 5 shows the proportion in which these fat sources are present in 2.84 grams of powder.

**Table 5**

| **Fat per 14 Calories (2.84 grams powder)** | |
|---|---|
| Total Fat, g | 1.0 |
| MCT Oil, g | 0.70 |
| Soybean Oil, g | 0.30 |
| Lecithin, g | 0.006 |
| Other, g | 0.024 |

In this particular embodiment, the carbohydrate sources are corn syrup solids and lactose. Tables 6 and 6A show alternative embodiments wherein the proportion in which those two carbohydrate sources are present in 2.84 grams of powder in each embodiment.

**Table 6**

| **Carbohydrate per 14 Calories (2.84 grams powder)** | |
|---|---|
| Total Carbohydrate, g | 0.228 |
| Corn Syrup Solids, g | 0.043 |
| Lactose, g | 0.005 |
| Others, g | 0.180 |

**Table 6A**

| **Carbohydrate per 14 Calories (2.84 grams powder)** | |
|---|---|
| Total Carbohydrate, g | 0.23 |
| Corn Syrup Solids, g | 0.043 |
| Lactose, g | 0.005 |
| Others, g | 0.182 |

Vitamins (vitamin A, vitamin D₃, vitamin E, vitamin K₁, thiamin, riboflavin, vitamin B₆ hydrochloride, vitamin B₁₂, niacinamide, folic acid, calcium pantothenate, biotin, ascorbic acid), and sources of minerals (calcium phosphate, calcium glycerophosphate, calcium gluconate, sodium citrate, potassium chloride, potassium citrate, potassium phosphate, magnesium phosphate, ferrous sulfate, zinc sulfate, cupric sulfate) are added to achieve the human milk fortifier compositions shown as alternative embodiments in Tables 7 and 7A which may be given to infants as a nutritional supplement to human milk.

**Table 7**

| **Components of 100.0 Kg. of Table 1 Nutrient Supplement** | | |
|---|---|---|
| Human Milk Fortifier Base | | |
| | MCT Oil | 24.711 kg |
| | Milk Protein Isolate, Low Lactose (Alapro 9405, from NZMP Co.) | 22.543 kg |
| | Hydrolyzed Whey Protein Isolate (BioZate-3, Davisco Foods) | 21.992 kg |
| | Soybean Oil | 10.590 kg |
| | Sodium Citrate Dihydrate, Powder | 1.249 kg |
| | Potassium Chloride | 1.015 kg |
| | Potassium Citrate | 0.922 kg |
| | Potassium Phosphate Monobasic | 0.553 kg |
| | Lecithin Concentrate | 0.210 kg |
| | Magnesium Phosphate Dibasic | 0.193 kg |
| Calciu'm Phosphate Tribasic, Ultrafine | | 4.753 kg |
| Calcium Glycerophosphate | | 4.369 kg |
| Calcium Gluconate Monohydrate | | 3.345 kg |
| | | |

| Dry Vitamin Premix for Human Milk Fortifier | | |
|---|---|---|
| | Ascorbic Acid | 746.592 g |
| | Tocopheryl Acetate, DL-Alpha, Dry, 50%, S.D. | 455.070 g |
| | Dry Vitamin A Palmitate Type 250-SD | 187.960 g |
| | Corn Syrup Solids | 182.878 g |
| | Niacinamide | 148.394 g |
| | Calcium Pantothenate | 40.434 g |
| | Vitamin K₁, Dry Phytonadione USP 1% | 18.868 g |
| | Vitamin D₃ Powder | 18.554 g |
| | Biotin, 1% Trituration | 13.361 g |
| | Riboflavin | 10.996 g |
| | Vitamin B₁₂, 0.1% in starch | 8.907 g |
| | Thiamin Hydrochloride | 7.725 g |
| | Pyridoxine Hydrochloride | 6.875 g |
| | Folic Acid | 1.386 g |
| | | |

| Iron Trituration | | |
|---|---|---|
| | Corn Syrup Solids | 1045.475 g |
| | Ferrous Sulfate, Heptahydrate | 269.800 g |
| | Ascorbic Acid | 33.725 g |
| | | |

| Trace Mineral Premix for Human Milk Fortifier | | |
|---|---|---|
| | Corn Syrup Solids | 349.414 g |
| | Zinc Sulfate, Monohydrate | 98.159 g |
| | Cupric Sulfate, Pentahydrate | 8.427 g |

**Table 7A**

| **Components of 100.0 Kg. of Table 1 Nutrient Supplement** | | |
|---|---|---|
| Human Milk Fortifier Base | | |
| | MCT Oil | 24.711 kg |
| | Milk Protein Isolate, Low Lactose (Alapro 9405, from NZMP Co.) | 22.543 kg |
| | Hydrolyzed Whey Protein Isolate (BioZate-3, Davisco Foods) | 21.992 kg |
| | Soybean Oil | 10.590 kg |
| | Sodium Citrate Dihydrate, Powder | 1.489 kg |
| | Potassium Chloride | 1.015 kg |
| | Potassium Citrate | 0.584 kg |
| | Potassium Phosphate Monobasic | 0.553 kg |
| | Lecithin Concentrate | 0.210 kg |
| | Magnesium Phosphate Dibasic | 0.193 kg |
| Calcium Phosphate Tribasic, Ultrafine | | 4.753 kg |
| Calcium Glycerophosphate | | 4.369 kg |
| Calcium Gluconate Monohydrate | | 3.345 kg |
| | | |

| Dry Vitamin Premix for Human Milk Fortifier | | |
|---|---|---|
| | Ascorbic Acid | 746.592 g |
| | Tocopheryl Acetate, DL-Alpha, Dry, 50%, S.D. | 455.070 g |
| | Dry Vitamin A Palmitate Type 250-SD | 187.960 g |
| | Corn Syrup Solids | 182.878 g |
| | Niacinamide | 148.394 g |
| | Calcium Pantothenate | 40.434 g |
| | Vitamin K₁, Dry Phytonadione USP 1 % | 18.868 g |
| | Vitamin D₃ Powder | 18.554 g |
| | Biotin, 1% Trituration | 13.361 g |
| | Riboflavin | 10.996 g |
| | Vitamin B₁₂, 0.1 % in starch | 8.907 g |
| | Thiamin Hydrochloride | 7.725 g |
| | Pyridoxine Hydrochloride | 6.875 g |
| | Folic Acid | 1.386 g |
| | | |

| Iron Trituration | | |
|---|---|---|
| | Corn Syrup Solids | 1045.475 g |
| | Ferrous Sulfate, Heptahydrate | 269.800 g |
| | Ascorbic Acid | 33.725 g |
| | | |

| Trace Mineral Premix for Human Milk Fortifier | | |
|---|---|---|
| | Corn Syrup Solids | 349.414 g |
| | Zinc Sulfate, Monohydrate | 98.159 g |
| | Cupric Sulfate, Pentahydrate | 8.427 g |

## Claims

1. The use of: (a) a fat component, and (b) a carbohydrate component, for the preparation of a human milk supplement for promoting the growth of a preterm infant, wherein said fat component is present in said supplement in an amount of 35% by dry weight or more, and said carbohydrate component is present in said supplement in an amount of 10% by dry weight or less.

2. The use of claim 1 wherein said fat component is present in said supplement in an amount of at least 39% by dry weight.

3. The use of claim 1 wherein said carbohydrate component is present in said supplement in an amount of no greater than 8% by dry weight.

4. The use of claim 1 wherein said fat component is present in said supplement in an amount of at least 39% by dry weight and said carbohydrate component is present in said supplement in an amount of no greater than 8% by dry weight.

5. The use of claim 1 wherein said fat component is present in said supplement in an amount of about 40% by dry weight and said carbohydrate component is present in said supplement in an amount of about 8% by dry weight.

6. The use of claim 1 wherein said supplement further comprises protein.

7. The use of claim 6 wherein said protein is present in said supplement in an amount of at least 39% by dry weight.

8. The use of claim 6 wherein said protein is present in said supplement in an amount of about 39% by dry weight.

9. The use of claim 1 wherein the caloric content of said supplement comprises greater than 62% fat and less than 5% carbohydrates.

10. The use of claim 1 wherein said fat component comprises medium chain triglycerides.

11. The use of claim 1 wherein said supplement further comprises protein chosen from the group consisting of whey and casein protein.

12. The use of claim 1 wherein said supplement further comprises protein and said fat component comprises medium chain triglycerides.

13. The use of any of claims 1 to 12 wherein said supplement is in dry form.

14. The use of any of claims 1 to 12 wherein said supplement is in liquid form.

15. A method of supplementing the nutritional value of human milk comprising adding to said human milk a supplement comprising: (a) 35% by dry weight or more of a fat component, and (b) 10% by dry weight or less of a carbohydrate component.

16. The method of claim 15 wherein said fat component is present in said supplement in an amount of at least 39% by dry weight.

17. The method of claim 15 wherein said carbohydrate component is present in said supplement in an amount of no greater than 8% by dry weight.

18. The method of claim 15 wherein said fat component is present in said supplement in an amount of at least 39% by dry weight and said carbohydrate component is present in said supplement in an amount of no greater than 8% by dry weight.

19. The method of claim 15 wherein said fat component is present in said supplement in an amount of about 40% by dry weight and said carbohydrate component is present in said supplement in an amount of about 8% by dry weight.

20. The method of claim 15 wherein said supplement further comprises protein.

21. The method of claim 20 wherein said protein is present in said supplement in an amount of at least 39% by dry weight.

22. The method of claim 20 wherein said protein is present in said supplement in an amount of about 39% by dry weight.

23. The method of claim 15 wherein the caloric content of said supplement comprises greater than 62% fat and less than 5% carbohydrates.

24. The method of claim 15 wherein said fat component comprises medium chain triglycerides.

25. The method of claim 15 wherein said supplement further comprises protein chosen from the group consisting of whey and casein protein.

26. The method of claim 15 wherein said supplement further comprises protein and said fat component comprises medium chain triglycerides.

27. The method of any of claims 15 to 26 wherein said supplement is in dry form.

28. The method of any of claims 15 to 26 wherein said supplement is in liquid form.

## Patentansprüche

1. Verwendung (a) einer Fettkomponente und (b) einer Kohlenhydratkomponente zur Herstellung eines Ergänzungsmittels für Milch des Menschen zur Förderung des Wachstums eines Frühgeborenen, wobei die Fettkomponente in dem Ergänzungsmittel in einer Menge von 35 % oder mehr, bezogen auf das Trockengewicht, vorhanden ist und die Kohlenhydratkomponente in dem Ergänzungsmittel in einer Menge von 10 % oder weniger, bezogen auf das Trockengewicht, vorhanden ist.

2. Verwendung nach Anspruch 1, wobei die Fettkomponente in dem Ergänzungsmittel in einer Menge von mindestens 39 %, bezogen auf das Trockengewicht, vorhanden ist.

3. Verwendung nach Anspruch 1, wobei die Kohlenhydratkomponente in dem Ergänzungsmittel in einer Menge von nicht höher als 8 %, bezogen auf das Trockengewicht, vorhanden ist.

4. Verwendung nach Anspruch 1, wobei die Fettkomponente in dem Ergänzungsmittel in einer Menge von mindestens 39 %, bezogen auf das Trockengewicht, vorhanden ist und die Kohlenhydratkomponente in dem Ergänzungsmittel in einer Menge von nicht höher als 8 %, bezogen auf das Trockengewicht, vorhanden ist.

5. Verwendung nach Anspruch 1, wobei die Fettkomponente in dem Ergänzungsmittel in einer Menge von etwa 40 %, bezogen auf das Trockengewicht, vorhanden ist und die Kohlenhydratkomponente in dem Ergänzungsmittel in einer Menge von etwa 8 %, bezogen auf das Trockengewicht, vorhanden ist.

6. Verwendung nach Anspruch 1, wobei das Ergänzungsmittel ferner Protein umfasst.

7. Verwendung nach Anspruch 6, wobei das Protein in dem Ergänzungsmittel in einer Menge von mindestens 39 %, bezogen auf das Trockengewicht, vorhanden ist.

8. Verwendung nach Anspruch 6, wobei das Protein in dem Ergänzungsmittel in einer Menge von etwa 39 %, bezogen auf das Trockengewicht, vorhanden ist.

9. Verwendung nach Anspruch 1, wobei der Kaloriengehalt des Ergänzungsmittels mehr als 62 % Fett und weniger als 5 % Kohlenhydrate umfasst.

10. Verwendung nach Anspruch 1, wobei die Fettkomponente Triglyceride mittlerer Kettenlänge umfasst.

11. Verwendung nach Anspruch 1, wobei das Ergänzungsmittel ferner Protein, welches aus der Gruppe ausgewählt ist, die aus Molke und Caseinprotein besteht, umfasst.

12. Verwendung nach Anspruch 1, wobei das Ergänzungsmittel ferner Protein umfasst und die Fettkomponente mittlere Triglyceride umfasst.

13. Verwendung nach einem der Ansprüche 1 bis 12, wobei das Ergänzungsmittel in trockener Form ist.

14. Verwendung nach einem der Ansprüche 1 bis 12, wobei das Ergänzungsmittel in flüssiger Form ist.

15. Verfahren zum Ergänzen des Nährwertes von Milch des Menschen, umfassend das Zugeben eines Ergänzungsmittels, umfassend (a) 35 % oder mehr, bezogen auf das Trockengewicht, einer Fettkomponente und (b) 10 % oder weniger, bezogen auf das Trockengewicht, einer Kohlenhydratkomponente, zu der Milch des Menschen.

16. Verfahren nach Anspruch 15, wobei die Fettkomponente in dem Ergänzungsmittel in einer Menge von mindestens 39 %, bezogen auf das Trockengewicht, vorhanden ist.

17. Verfahren nach Anspruch 15, wobei die Kohlenhydratkomponente in dem Ergänzungsmittel in einer Menge von nicht höher als 8 %, bezogen auf das Trockengewicht, vorhanden ist.

18. Verfahren nach Anspruch 15, wobei die Fettkomponente in dem Ergänzungsmittel in einer Menge von mindestens 39 %, bezogen auf das Trockengewicht, vorhanden ist und die Kohlenhydratkomponente in dem Ergänzungsmittel in einer Menge von nicht höher als 8 %, bezogen auf das Trockengewicht, vorhanden ist.

19. Verfahren nach Anspruch 15, wobei die Fettkomponente in dem Ergänzungsmittel in einer Menge von etwa 40 %, bezogen auf das Trockengewicht, vorhanden ist und die Kohlenhydratkomponente in dem Ergänzungsmittel in einer Menge von etwa 8 %, bezogen auf das Trockengewicht, vorhanden ist.

20. Verfahren nach Anspruch 15, wobei das Ergänzungsmittel ferner Protein umfasst.

21. Verfahren nach Anspruch 20, wobei das Protein in dem Ergänzungsmittel in einer Menge von mindestens 39 %, bezogen auf das Trockengewicht, vorhanden ist.

22. Verfahren nach Anspruch 20, wobei das Protein in dem Ergänzungsmittel in einer Menge von etwa 39 %, bezogen auf das Trockengewicht, vorhanden ist.

23. Verfahren nach Anspruch 15, wobei der Kaloriengehalt des Ergänzungsmittels mehr als 62 % Fett und weniger als 5 % Kohlenhydrate umfasst.

24. Verfahren nach Anspruch 15, wobei die Fettkomponente Triglyceride mittlerer Kettenlänge umfasst.

25. Verfahren nach Anspruch 15, wobei das Ergänzungsmittel ferner Protein, welches aus der Gruppe ausgewählt ist, die aus Molke und Caseinprotein besteht, umfasst.

26. Verfahren nach Anspruch 15, wobei das Ergänzungsmittel ferner Protein umfasst und die Fettkomponente Triglyceride mittlerer Kettenlänge umfasst.

27. Verfahren nach einem der Ansprüche 15 bis 26, wobei das Ergänzungsmittel in trockener Form ist.

28. Verfahren nach einem der Ansprüche 15 bis 26, wobei das Ergänzungsmittel in flüssiger Form ist.

## Revendications

1. Utilisation : (a) d'un composant de matière grasse, et (b) d'un composant d'hydrate de carbone, pour la préparation d'un supplément pour le lait humain destiné à promouvoir la croissance d'un enfant prématuré, dans laquelle ledit composant de matière grasse est présent dans ledit supplément en une quantité de 35 % en poids sec ou plus, et ledit composant d'hydrate de carbone est présent dans ledit supplément en une quantité de 10 % en poids sec ou moins.

2. Utilisation selon la revendication 1, dans laquelle ledit composant de matière grasse est présent dans ledit supplément en une quantité d'au moins 39 % en poids sec.

3. Utilisation selon la revendication 1, dans laquelle ledit composant d'hydrate de carbone est présent dans ledit supplément en une quantité pas supérieure à 8 % en poids sec.

4. Utilisation selon la revendication 1, dans laquelle ledit composant de matière grasse est présent dans ledit supplément en une quantité d'au moins 39 % en poids sec et ledit composant d'hydrate de carbone est présent dans ledit supplément en une quantité pas supérieure à 8 % en poids sec.

5. Utilisation selon la revendication 1, dans laquelle ledit composant de matière grasse est présent dans ledit supplément en une quantité d'environ 40 % en poids sec et ledit composant d'hydrate de carbone est présent dans ledit supplément en une quantité d'environ 8 % en poids sec.

6. Utilisation selon la revendication 1, dans laquelle ledit supplément comprend en outre une protéine.

7. Utilisation selon la revendication 6, dans laquelle ladite protéine est présente dans ledit supplément en une quantité d'au moins 39 % en poids sec.

8. Utilisation selon la revendication 6, dans laquelle ladite protéine est présente dans ledit supplément en une quantité d'environ 39 % en poids sec.

9. Utilisation selon la revendication 1, dans laquelle la teneur calorique dudit supplément comprend plus de 62 % de matière grasse et moins de 5 % d'hydrates de carbone.

10. Utilisation selon la revendication 1, dans laquelle ledit composant de matière grasse comprend des triglycérides à chaîne moyenne.

11. Utilisation selon la revendication 1, dans laquelle ledit supplément comprend en outre une protéine choisie dans le groupe constitué par la protéine de lactosérum et de caséine.

12. Utilisation selon la revendication 1, dans laquelle ledit supplément comprend en outre une protéine et ledit composant de matière grasse comprend des triglycérides à chaîne moyenne.

13. Utilisation selon l'une quelconque des revendications 1 à 12, dans laquelle ledit supplément se trouve sous une forme anhydre.

14. Utilisation selon l'une quelconque des revendications 1 à 12, dans laquelle ledit supplément se trouve sous une forme liquide.

15. Procédé de supplémentation de la valeur nutritive du lait humain comprenant l'ajout audit lait humain d'un supplément comprenant (a) 35 % en poids sec ou plus d'un composant de matière grasse, et (b) 10 % en poids sec ou moins d'un composant d'hydrate de carbone.

16. Procédé selon la revendication 15, dans lequel ledit composant de matière grasse est présent dans ledit supplément en une quantité d'au moins 39 % en poids sec.

17. Procédé selon la revendication 15, dans lequel ledit composant d'hydrate de carbone est présent dans ledit supplément en une quantité pas supérieure à 8 % en poids sec.

18. Procédé selon la revendication 15, dans lequel ledit composant de matière grasse est présent dans ledit supplément en une quantité d'au moins 39 % en poids sec et ledit composant d'hydrate de carbone est présent dans ledit supplément en une quantité pas supérieure à 8 % en poids sec.

19. Procédé selon la revendication 15, dans lequel ledit composant de matière grasse est présent dans ledit supplément en une quantité d'environ 40 % en poids sec et ledit composant d'hydrate de carbone est présent dans ledit supplément en une quantité d'environ 8 % en poids sec.

20. Procédé selon la revendication 15, dans lequel ledit supplément comprend en outre une protéine.

21. Procédé selon la revendication 20, dans lequel ladite protéine est présente dans ledit supplément en une quantité d'au moins 39 % en poids sec.

22. Procédé selon la revendication 20, dans lequel ladite protéine est présente dans ledit supplément en une quantité d'environ 39 % en poids sec.

23. Procédé selon la revendication 15, dans lequel la teneur calorique dudit supplément comprend plus de 62 % de matière grasse et moins de 5 % d'hydrates de carbone.

24. Procédé selon la revendication 15, dans lequel ledit composant de matière grasse comprend des triglycérides à chaîne moyenne.

25. Procédé selon la revendication 15, dans lequel ledit supplément comprend en outre une protéine choisie dans le groupe constitué par la protéine de lactosérum et de caséine.

26. Procédé selon la revendication 15, dans lequel ledit supplément comprend en outre une protéine et ledit composant de matière grasse comprend des triglycérides à chaîne moyenne.

27. Procédé de l'une quelconque des revendications 15 à 26, dans lequel ledit supplément se trouve sous une forme anhydre.

28. Procédé de l'une quelconque des revendications 15 à 26, dans lequel ledit supplément se trouve sous une forme liquide.
